# EUROPÄISCHE PATENTSCHRIFT

(11) **EP 1 084 678 B1**
(45) Veröffentlichungstag und Bekanntmachung des Hinweises auf die Patenterteilung: **07.12.2005**
(21) Anmeldenummer: 00203157.3
(22) Anmeldetag: 12.09.2000
(51) Int. Cl.: A61B 6/10

(54) **Röntgeneinrichtung mit Indikatormitteln**
X-ray device with indicating means
Appareil de radiographie avec moyens indicateurs

(30) Priorität: 14.09.1999 DE 19943898
(43) Veröffentlichungstag der Anmeldung: 21.03.2001
(73) Patentinhaber: Philips Intellectual Property & Standards GmbH, 20099 Hamburg (DE); Koninklijke Philips Electronics N.V., 5621 BA Eindhoven (NL)
(72) Erfinder: Köhler, Thomas, Philips Corporate I.P. GmbH, 52064 Aachen (DE); Rasche, Volker, Philips Corporate I.P. GmbH, 52064 Aachen (DE); Sabczynski, Jörg, Philips Corporate I.P. GmbH, 52064 Aachen (DE)
(74) Vertreter: Volmer, Georg

(56) Entgegenhaltungen:
- EP-A- 0 819 407
- JP-A- 7 047 063
- JP-A- 8 266 535
- JP-A- 8 336 518

## Beschreibung

Die Erfindung betrifft eine Röntgeneinrichtung, insbesondere eine Fluoroskopie-Röntgeneinrichtung, mit einer Röntgenquelle und einem Röntgendetektor zur kontinuierlichen Erstellung von Röntgenbildern eines Patienten aus unveränderter Position von Röntgenquelle und Röntgendetektor.

Derartige Röntgeneinrichtungen werden häufig als Operationshilfe verwendet, um operative Eingriffe an einem Patienten überwachen zu können. Dazu werden aus fester Position von Röntgenquelle und Röntgendetektor kontinuierlich Röntgenbilder des interessierenden Untersuchungsbereichs erstellt und on-line auf einem Monitor dargestellt, so daß der behandelnde Arzt seinen Eingriff unmittelbar verfolgen kann. Dabei tritt jedoch das Problem auf, daß der behandelnde Arzt mit Körperteilen, insbesondere mit seinen Händen, in den von der unsichtbaren Röntgenstrahlung durchsetzten Bereich zwischen Patient und Röntgenquelle bzw. Röntgendetektor ungewollt und unbemerkt hineinfaßt und dadurch ebenfalls eine Röntgendosis erhält. Trotz der verhältnismäßig geringen Dosis bei einmaligem Hineinfassen in den Röntgenstrahl kann dies auf lange Sicht bei häufigem Vorkommen zu einer Schädigung der Hände des Arztes führen.

Aus der US 5,873,826 ist eine Computer-Tomographie-Einrichtung mit einer rotierenden Röntgenquelle und einem rotierenden Röntgendetektor bekannt, welche unmittelbar neben der Röntgenquelle eine ebenfalls rotierende Lichtquelle aufweist. Um dort zu verhindern, daß die Hände des behandelnden Arztes einer zu hohen Röntgendosis ausgesetzt werden, ist vorgesehen, in einem ersten Winkelbereich, in dem die Hände des Arztes normalerweise arbeiten, die Röntgenstrahlung abzuschalten und die Röntgenstrahlung nur in einem zweiten (dem restlichen) Winkelbereich anzuschalten. Um dem Arzt zu verdeutlichen, in welchem Winkelbereich die Röntgenstrahlung abgeschaltet ist, wird dieser Winkelbereich mittels der Lichtquelle erleuchtet, während im übrigen Winkelbereich die Lichtquelle ausgeschaltet ist (oder umgekehrt). Durch die Abschaltung der Röntgenquelle in dem ersten Winkelbereich wird zudem erreicht, daß die Qualität der Röntgenbilder nicht durch die Hände des Arztes verschlechtert wird.

Aus der EP-A-0 819 407 ist eine Röntgeneinrichtung bekannt, die Indikatormitteln zur Ausleuchtung des Bereiches zwischen Röntgenquelle und Patient aufweist.

Der vorliegenden Erfindung liegt die Aufgabe zugrunde, eine eingangs genannte Röntgeneinrichtung durch Maßnahmen zum Schutze der behandelnden Personen zu verbessern.

Diese Aufgabe wird durch eine Röntgeneinrichtung gemäß Anspruch 1 gelöst.

Bei der aus der US 5,873,826 bekannten CT-Einrichtung ist nur der Bereich zwischen Röntgenquelle und Patient in einem bestimmten Winkelbereich für den Arzt sichtbar und vor Röntgenstrahlungen geschützt ist, der Bereich zwischen Röntgendetektor und Patient dagegen ist, auch wenn sich der Röntgendetektor oberhalb des Patienten befindet und die Hand des Arztes zwischen Detektor und Patient arbeitet, nicht sichtbar und geschützt. Bei der erfindungsgemäßen Röntgeneinrichtung wird demgegenüber auf den Bereich oberhalb des Patienten unabhängig von der Stellung der Röntgenquelle und des Röntgendetektors abgestellt, da sich dort normalerweise die Hände des Arztes bei einer Behandlung befinden. Bei einer C-Bogen-Röntgeneinrichtung beispielsweise befinden sich die Röntgenquelle in einer Stellung unterhalb des Patienten und der Röntgendetektor oberhalb des Patienten, so daß die Indikatormittel bevorzugt bei einer solchen Röntgeneinrichtung am Röntgendetektor angebracht sind, um den Strahlungsbereich zwischen Röntgendetektor und Patient auszuleuchten bzw. zu überwachen. Es wird bei der erfindungsgemäßen Röntgeneinrichtung auch keine Abschaltung der Röntgenquelle vorgesehen in einem bestimmten Winkelbereich oder wenn sich die Hand des Arztes im Strahlungsbereich befindet, da ja dann bei einer Röntgenbilderstellung aus fester Position von Röntgenquelle und Röntgendetektor keine aktuellen Röntgenbilder verfügbar und somit eine Überwachung des Eingriffs des Arztes nicht möglich wären. Es bleibt vielmehr der eigenen Verantwortung des Arztes überlassen, ob er mit seinen Händen in den ausgeleuchteten und/oder überwachten Strahlungsbereich hineinfassen will; bei der erfindungsgemäßen Röntgeneinrichtung wird ihm jedoch unmittelbar mitgeteilt, wenn er dies tut.

Vorteilhafte Ausgestaltungen ergeben sich insbesondere aus den weiteren Ansprüchen. Bevorzugt sind die Indikatormittel als optische Anzeigemittel quasi zur Sichtbarmachung des Röntgenstrahlung durchsetzten Bereiches, als optische Überwachungsmittel zur Überwachung eines Eingriffs in den Strahlungsbereich und/oder als akustische Warnmittel zur Abgabe eines akustischen Warnsignals bei Eingriff in den Strahlungsbereich ausgestaltet.

Die Erfindung wird nachfolgend anhand der Zeichnung näher erläutert.
Es zeigen:
- Fig. 1: eine erfindungsgemäße Fluoroskopie-Röntgeneinrichtung während eines Eingriffs,
- Fig. 2: eine Draufsicht auf einen Röntgendetektor mit Indikatormitteln und
- Fig. 3: eine weitere Ausgestaltung einer erfindungsgemäßen Fluoroskopie-Röntgeneinrichtung.

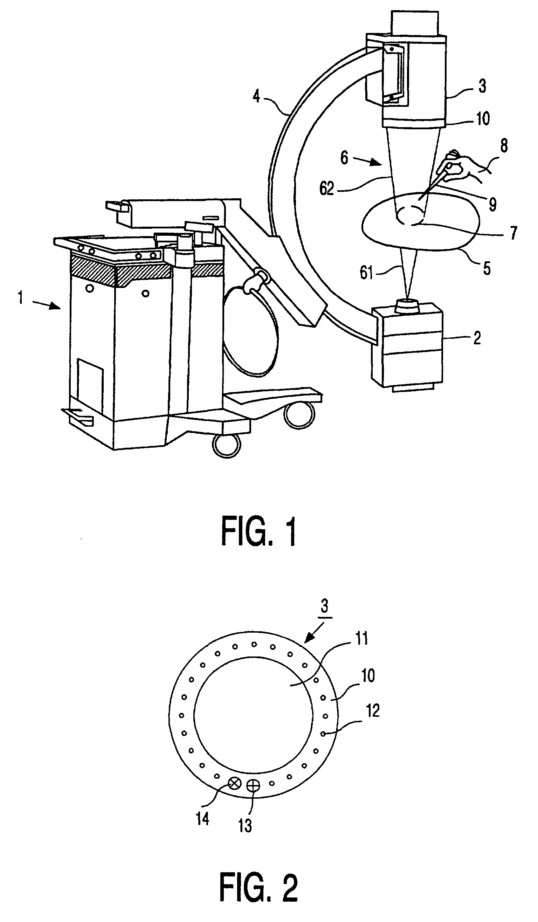

Die in Fig. 1 dargestellte Fluoroskopie-Röntgeneinrichtung 1 weist eine Röntgenquelle 2 und einen Röntgendetektor 3 auf, die beide an einem C-Bogen 4 angeordnet sind. In der gezeigten normalen Betriebsstellung befindet sich die Röntgenquelle 2 unterhalb eines symbolisch dargestellten Patienten 5 und der Röntgendetektor 3 oberhalb des Patienten 5. Mit 6 ist die einen kegelförmigen Röntgenstrahl (Strahl 61 unterhalb des Patienten 5, Strahl 62 oberhalb des Patienten 5) bildende Röntgenstrahlung gezeigt, die den Patienten 5 im Untersuchungsbereich 7 durchtritt. Weiterhin ist symbolisch die Hand 8 eines Arztes gezeigt, der mit einem medizinischen Instrument 9, beispielsweise einem Skalpell oder einer Biopsienadel einen Eingriff am Patienten 5 vornimmt und diesen auf einem nicht dargestellten Monitor unmittelbar verfolgen möchte, wozu mittels der Röntgeneinrichtung 1 kontinuierlich Röntgenbilder erstellt werden. An der zu dem Patient 5 hinweisenden Unterseite des Röntgendetektors 3 sind außerdem Indikatormittel 10 angeordnet, die für den Arzt den von Röntgenstrahlung durchsetzten Strahlungsbereich 62 zwischen Röntgendetektor 3 und Patient 5 sichtbar machen bzw. überwachen sollen.

Wie in einer Draufsicht auf die Unterseite des Röntgendetektors 3 in Fig. 2 näher gezeigt ist, weisen die Indikatormittel 10 mehrere Laserdioden 12 auf, die ringförmig um die Detektorfläche 11 angeordnet sind. Die Strahlungsrichtung dieser Laserdioden 12 ist auf den Fokus der Röntgenröhre 2 ausgerichtet, so daß im wesentlichen die Umrisse des Röntgenstrahles 62 ausgeleuchtet werden. Außerdem sind in der gezeigten Ausführungsform eine Infrarot-Leuchtdiode 13 und ein Infrarotdetektor 14 vorgesehen, die den beschriebenen Strahlungsbereich 62 nach dem Prinzip eines Bewegungsmelders überwachen und ein Signal abgeben, wenn die Hand 8 des Arztes oder ein Instrument 9 in den Strahlungsbereich 62 hineinkommt.

Anstelle mehrerer Laserdioden 12 können auch andere Leuchtmittel eingesetzt werden, wie beispielsweise eine einzelne Laserdiode mit mehreren Lichtstrahlen, mehrere Lichtwellenleiter oder eine UV-Strahlungsvorrichtung, die den Strahlungsbereich 62 mit ultraviolettem Licht ausstrahlt, wobei der Arzt dann fluoreszierende Handschuhe tragen oder die Behandlungsinstrumente fluoreszierend ausgestaltet werden müßten. Wesentlich dabei ist jedoch immer, daß die Indikatormittel auf den Fokus der Röntgenröhre 2 ausgerichtet sind und somit möglichst genau den Strahlungsbereich 62 ausleuchten bzw. überwachen und daß der Arzt unmittelbar bemerken kann, daß er in den Strahlungsbereich 62 hineinfaßt, sei es dadurch, daß er es direkt sieht, oder dadurch, daß ein zusätzliches optisches oder akustischen Warnsignal gegeben wird. Auch die Anzahl und die Anordnung der in Fig. 2 dargestellten Laserdioden 12 bzw. der IR-Diode 13 und des IR-Detektors 14 ist lediglich beispielhaft und kann je nach Art und Leistung der verwendeten Indikatormittel bzw. der damit auszurüstenden Röntgeneinrichtung entsprechend ausgewählt werden.

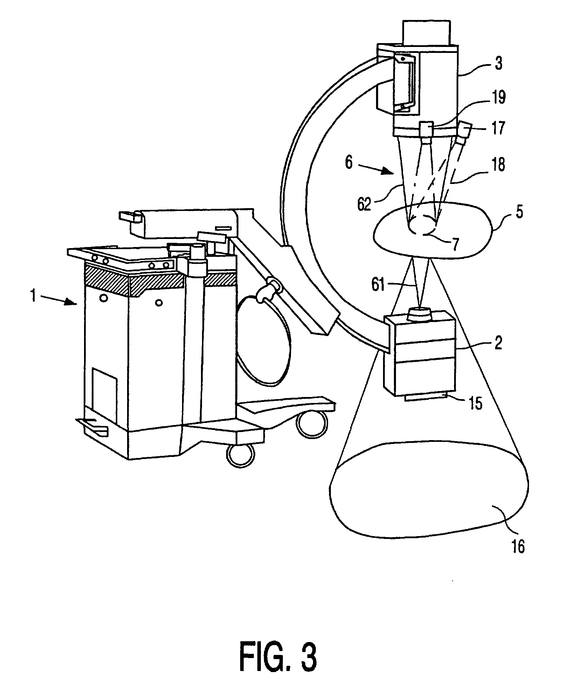

In Fig. 3 ist eine weitere Ausführungsform einer erfindungsgemäßen Fluoroskopie-Röntgeneinrichtung gezeigt. Dort wird als Indikatormittel eine Videokamera 17 verwendet, die im wesentlichen einen Überwachungsbereich 18 optisch überwacht, der zwar nicht identisch ist mit dem Röntgenstrahlungsbereich 62, jedoch immer mehr deckungsgleich mit diesem ist, je näher die Bereiche dem Patienten 5 kommen, und die sich im Untersuchungsbereich 7 in etwa decken. Diese Videokamera 17 ist beispielsweise derart ausgestaltet, daß sie Bewegungen im Überwachungsbereich 18 erkennen und ein entsprechendes Signal abgeben kann oder daß auf dem Röntgenmonitor oder einem separaten Monitor das von der Videokamera 17 aufgenommene Bild eingeblendet wird. Zusätzlich ist in dieser Ausführungsform eine weitere Kamera 19 an der Detektoranordnung 3 angeordnet, die jeweils das aktuelle Röntgenbild auf die Oberfläche des Patienten 5 im Untersuchungsbereich 7 projizieren kann, was ebenfalls dem Arzt zur Operationshilfe dient.

Bei den gezeigten Röntgeneinrichtungen tritt Röntgenstrahlung im wesentlichen in einer Hauptrichtung aus der Röntgenquelle 2 aus, in den Figuren 1 und 3 also in vertikaler Richtung zum Detektor hin. Eine weitere nicht unbeachtliche Streustrahlung (wird im Patienten 5 erzeugt und vorwiegend unter 180° zur Hauptrichtung abgestrahlt, also in den genannten Figuren vom Körper 5 ausgehend in Richtung der Röntgenquelle 2). Auch diese Röntgenstrahlung kann bei häufiger Bestrahlung zu einer Schädigung des Körpers, in der gezeigten Stellung der Röntgenquelle der Füße des Arztes führen, sofern sie nicht von der Röntgenquelle abgeschattet wird. Um auch diesen bevorzugten Streustrahlungsbereich für den Arzt sichtbar zu machen bzw. diesen zu überwachen, ist bei der in Fig. 3 gezeigten Ausführungsform an der Unterseite der Röntgenquelle 2 ein Indikatormittel 15 mit einem Ausleuchtungs-und/oder Überwachungsbereich 16 angeordnet. Dieses Indikatormittel 15 kann ebenso ausgestaltet sein wie die bereits erwähnten Indikatormittel, wobei der Ausleuchtungs- bzw. Überwachungsbereich möglichst dem Bereich mit der höchsten Streustrahlung in dieser Richtung von der Röntgenquelle 2 aus gesehen entsprechen soll. Damit kann der Arzt erkennen, in welchem Bereich bevorzugt Streustrahlung auftritt, oder er erhält eine Warnung, falls er sich mit Körperteilen, beispielsweise seinen Füßen, in diesem Streustrahlungsbereich aufhält.

Die Erfindung ist nicht auf die Verwendung bei der gezeigten Fluoroskopie-Röntgeneinrichtung mit einem C-Bogen beschränkt, sondern ist grundsätzlich bei jeder Röntgeneinrichtung einsetzbar, also beispielsweise auch bei einer fest installierten Röntgeneinrichtung. Weiterhin können die Indikatormittel statt am Röntgendetektor auch an der Röntgenquelle oder an beiden Bauteilen angebracht sein, insbesondere dann, wenn die Röntgenquelle oberhalb des Patienten angeordnet ist. Es können auch mehrere verschiedene Indikatormittel miteinander kombiniert werden, um die Sicherheit für den Arzt zu erhöhen.

Außerdem kann vorgesehen werden, daß die Indikatormittel an die Geometrie des Röntgenstrahles, insbesondere an die Größe des Öffnungswinkels der Röntgenstrahlung anpaßbar ausgestaltet sind. Dazu kann beispielsweise vorgesehen sein, daß ein Signal zur Veränderung der Blendenöffnung einer an der Röntgenquelle angebrachten Blende, wodurch die Strahlgeometrie verändert wird, auch an die Indikatormittel weitergegeben wird, damit diese so ausgerichtet werden können, daß deren Ausleuchtungs- bzw. Überwachungsbereich dem veränderten Strahlungsbereich angepaßt wird. Die Indikatormittel könnten für den Fall, daß sie den Strahlungsbereich quasi sichtbar machen, außerdem dazu benutzt werden, vor der Erstellung von Röntgenbildern am Patienten zu zeigen, welches der durchstrahlte Untersuchungsbereich sein wird und somit dem Arzt eine Anpassung und Optimierung dieses durchstrahlten Bereiches ermöglichen, ohne daß dazu die Röntgenstrahlung eingeschaltet sein muß.

## Patentansprüche

1. Röntgeneinrichtung, insbesondere Fluoroskopie-Röntgeneinrichtung (1),
- mit einer Röntgenquelle (2) und einem Röntgendetektor (3) zur kontinuierlichen Erstellung von Röntgenbildern eines Patienten (5) aus unveränderter Position von Röntgenquelle (2) und Röntgendetektor (3) und
- mit Indikatormitteln (10, 11; 15, 17), die dazu vorgesehen sind, einen im wesentlichen von Röntgenstrahlung (62) durchsetzten Strahlungsbereich, der oberhalb des Patienten (5) und zwischen dem Röntgendetektor (3) und dem Patienten (5) liegt, auszuleuchten, und/oder mit Indikatormitteln (10, 11; 15, 17), die dazu vorgesehen sind, einen im wesentlichen von Röntgenstrahlung (62) durchsetzten Strahlungsbereich oberhalb des Patienten (5) zu überwachen.

2. Röntgeneinrichtung nach Anspruch 1,
**dadurch gekennzeichnet, daß** die Indikatormittel (10, 11; 15, 17) am Röntgendetektor (3) und/oder an der Röntgenquelle (2) angeordnet sind.

3. Röntgeneinrichtung nach einem der vorherigen Ansprüche,
**dadurch gekennzeichnet, daß** die Indikatormittel (10, 11; 15, 17) optische, im wesentlichen den von Röntgenstrahlung durchsetzten Strahlungsbereich oberhalb des Patienten ausleuchtenden Leuchtmittel (12), insbesondere mehrere Laserdioden, Lichtwellenleiter oder eine UV-Strahlungsvorrichtung aufweisen.

4. Röntgeneinrichtung nach einem der vorherigen Ansprüche,
**dadurch gekennzeichnet, daß** die Indikatormittel (10, 11; 15, 17) eine Vorrichtung (19) aufweisen zur Projektion des aktuellen Röntgenbildes auf die von Röntgenstrahlung durchsetzte Oberfläche des Patienten (5).

5. Röntgeneinrichtung nach einem der vorherigen Ansprüche,
**dadurch gekennzeichnet, daß** die Indikatormittel (10, 11; 15, 17) optische Überwachungsmittel (13, 14; 15, 17) aufweisen, insbesondere einen Infrarotsender (13) und einen Infrarotdetektor (14) oder eine Videokamera (15, 17) zur Überwachung im wesentlichen des von Röntgenstrahlung durchsetzten Strahlungsbereichs oberhalb des Patienten.

6. Röntgeneinrichtung nach Anspruch 5,
**dadurch gekennzeichnet, daß** die Indikatormittel (10, 11; 15, 17) eine akustische Warnvorrichtung aufweisen zur Abgabe eines akustischen Warnsignals bei Einbringen eines Objekts (8, 9) in den überwachten Strahlungsbereich (62; 16).

7. Röntgeneinrichtung nach einem der vorherigen Ansprüche,
**dadurch gekennzeichnet, daß** weitere Indikatormittel (15) derart an der Röntgenquelle (2) angeordnet sind, daß im wesentlichen der von in entgegengesetzer Richtung zur Hauptrichtung aus der Röntgenquelle austretender Röntgenstrahlung durchsetzte Bereich (16) ausgeleuchtet und/oder überwacht wird.

8. Röntgeneinrichtung nach einem der vorherigen Ansprüche,
**dadurch gekennzeichnet, daß** der Ausleuchtungs- bzw. Überwachungsbereich der Indikatormittel (10, 11; 15, 17) an die Strahlgeometrie, insbesondere den Öffnungswinkel der Röntgenstrahlung (6) anpaßbar ist.

## Claims

1. An X-ray device, notably a device for X-ray fluoroscopy (1), which includes
- an X-ray source (2) and an X-ray detector (3) for the continuous formation of X-ray images of a patient (5) from an invariable position of the X-ray source (2) and X-ray detector (3), and
- indicator means (10, 11; 15, 17) for illuminating a radiation zone in essence traversed by X-rays (62), which is situated over the patient (5) and between the X-ray detector (3) and the patient (5), and/or indicator means (10, 11; 15, 17), which are provided for monitoring a radiation zone over the patient, which is traversed in essence by X-rays (62).

2. An X-ray device as claimed in claim 1, **characterized in that** the indicator means (10, 11; 15, 17) are arranged on the X-ray detector (3) and/or on the X-ray source (2).

3. An X-ray device as claimed in one of the preceding claims, **characterized in that** the indicator means (10, 11; 15, 17) include optical illumination means (12), notably a plurality of laser diodes, optical conductors or a UV radiation device, which are arranged in essence to illuminate the radiation zone over the patient which is traversed by X-rays.

4. An X-ray device as claimed in one of the preceding claims, **characterized in that** the indicator means (10, 11; 15, 17) include a device (19) for projecting the current X-ray image onto the surface of the patient (5) which is traversed by X-rays.

5. An X-ray device as claimed in one of the preceding claims, **characterized in that** the indicator means (10, 11; 15, 17) include optical monitoring means (13, 14; 15, 17), notably an infrared transmitter (13) and an infrared detector (14) or a video camera (15, 17), for monitoring essentially the radiation zone over the patient which is traversed by X-rays.

6. An X-ray device as claimed in claim 5, **characterized in that** the indicator means (10, 11; 15, 17) include an acoustic alarm device for producing an acoustic alarm signal when an object (8, 9) enters the monitored radiation zone (62; 16).

7. An X-ray device as claimed in one of the preceding claims, **characterized in that** further indicator means (15) are arranged on the X-ray source (2) in such a manner that essentially the zone (16) which is traversed by X-rays from the X-ray source in the direction opposing the main direction is illuminated and/or monitored.

8. An X-ray device as claimed in one of the preceding claims, **characterized in that** the illumination or monitoring zone of the indicator means (10, 11; 15, 17) can be adapted to the beam geometry, notably to the angle of aperture of the X-rays (6).

## Revendications

1. Appareil de radiographie, en particulier appareil de radiographie à fluoroscopie (1),
- avec une source de rayons X (2) et un détecteur de rayons X (3) pour l'élaboration continue de clichés radiographiques d'un patient (5) à partir d'une position inchangée de la source de rayons X (2) et du détecteur de rayons X (3), et
- avec des moyens indicateurs (10, 11; 15, 17) qui sont prévus pour exposer une zone de rayonnement essentiellement traversée par les rayons X (62) qui se trouve au-dessus du patient (5) et entre le détecteur de rayons X (3) et le patient (5) et/ou avec des moyens indicateurs (10, 11; 15 17) qui sont prévus pour surveiller une zone de rayonnement essentiellement traversée par des rayons X (62) au-dessus du patient (5).

2. Appareil de radiographie selon l'une des revendications précédentes, **caractérisé en ce que** les moyens indicateurs (10,11; 15,17) sont disposés sur le détecteur de rayon X (3) et/ou sur la source de rayons X (2).

3. Appareil de radiographie selon l'une des revendications précédentes, **caractérisé en ce que** les moyens indicateurs (10, 11; 15, 17) présentent des moyens luminescents (12) optiques exposant essentiellement la zone de rayonnement traversée par les rayons X au-dessus du patient, en particulier plusieurs diodes laser, guides d'ondes lumineux ou un dispositif de rayonnement U.V.

4. Appareil de radiographie selon l'une des revendications précédentes, **caractérisé en ce que** les moyens indicateurs (10, 11; 15, 17) présentent un dispositif (19) pour la projection de l'image radiographique actuelle sur la surface du patient (5) traversée par les rayons X.

5. Appareil de radiographie selon l'une des revendications précédentes, **caractérisé en ce que** les moyens indicateurs (10, 11; 15, 17) présentent des moyens de surveillance optiques (13, 14; 15; 17), en particulier un émetteur infrarouges (13) et un détecteur infrarouge (14) ou une caméra vidéo (15, 17) pour la surveillance essentiellement de la zone de rayonnement traversée par les rayons X au-dessus du patient.

6. Appareil de radiographie selon la revendication 5, **caractérisé en ce que** les moyens indicateurs (10, 11; 15, 17) présentent un dispositif d'avertissement acoustique pour la délivrance d'un signal d'avertissement acoustique en cas d'introduction d'un objet (8, 9) dans la zone de rayonnement surveillée (62, 16).

7. Appareil de radiographie selon l'une des revendications précédentes, **caractérisé en ce que** les autres moyens indicateurs (15) sont disposés sur la source de rayons X (2) de telle sorte que la zone (16) traversée par des rayons X sortant de la source de rayons X dans la direction opposée de la direction principale soit exposée et/ou surveillée.

8. Appareil de radiographie selon l'une des revendications précédentes, **caractérisé en ce que** la zone d'exposition ou de surveillance des moyens indicateurs (10, 11; 15, 17) peut être adaptée à la géométrie des rayons, en particulier à l'angle d'ouverture des rayons X (6).
